# EUROPEAN PATENT APPLICATION

(11) **EP 1 241 181 A1**
(43) Date of publication of application: **18.09.2002**
(21) Application number: 00967909.3
(22) Date of filing: 04.10.2000
(51) Int. Cl.: C07K 14/705, C12N 5/16, C12N 15/12, A61K 38/17

(54) **CD29 PIG PROTEIN, NUCLEIC ACID CODING FOR SAID PROTEIN AND THE APPLICATIONS THEREOF**

(30) Priority: 05.10.1999 ES 9902193
(71) Applicant: Biovet-Uco, S.L., 14071 Cordoba (ES)
(72) Inventor: GARRIDO PAVON, Juan José, E-14711 Encinarejo de Cordoba (ES); LLANES RUIZ, Diego, E-14011 Cordoba (ES); BABANCHO MEDINA, Manuel, E-14002 Cordoba (ES); JIMENEZ MARIN, Angeles Maria, E-14004 Cordoba (ES)
(74) Representative: Maldonado Jordan, Julia
(86) International application number: ES0000374
(87) International publication number: WO01025279

(57) **Abstract**

CD29 native pig protein contains epitopes Gal-α-(1,3)-Gal that are recognized by xenoreactive natural human antibodies unleashing hyperacute rejection of the xenograft. The invention relates to proteins based on CD29 pig protein having an amino acid sequence similar to that of CD29 native protein or a modified amino acid sequence with an identical, reduced or zero expression of epitope Gal-α-(1,3)-Gal, to nucleic acid molecules coding for said proteins and to the utilization of said proteins and nucleic acid molecules in the development of strategies to reduce or prevent hyperacute rejection associated with xenotransplant therapies.

SEQUENCE LISTING

<110>     BIOVET-UCO, S.L.

<120>     CD29 PIG PROTEIN, NUCLEIC ACID CODING FOR SAID PROTEIN
          AND THE APPLICATIONS THEREOF

<140>     PCT/ES00/00374
<141>     2000-10-04

<150>     P9902193
<151>     1999-10-05

<160>     4

<170>     PatentIn version 2.0

<210>     1
<211>     798
<212>     PRT
<213>     Pig

<400>     1
Met Asn Leu Gln Leu Ile Phe Trp Ile Gly Leu Ile Ser Ser Val Cys
 1               5                   10                  15

Tyr Val Phe Gly Gln Ala Asp Glu Asn Arg Cys Leu Lys Ala Asn Ala
            20                  25                  30

Lys Ser Cys Gly Glu Cys Ile Gln Ala Gly Pro Asn Cys Gly Trp Cys
        35                  40                  45

Thr Asn Ser Thr Phe Leu Gln Glu Gly Met Pro Thr Ser Ala Arg Cys
    50                  55                  60

Asp Asp Leu Glu Ala Leu Arg Lys Lys Gly Cys His Pro Asp Asp Ile
65                  70                  75                  80

Glu Asn Pro Arg Gly Ser Lys Asn Ile Lys Lys Asn Lys Asn Val Thr
                85                  90                  95

Asn Arg Ser Lys Gly Thr Ala Glu Lys Leu Gln Pro Glu Asp Ile Thr
            100                 105                 110

Gln Ile Gln Pro Gln Gln Leu Val Leu Gln Leu Arg Ser Gly Glu Pro
        115                 120                 125

Gln Thr Phe Thr Leu Lys Phe Lys Arg Ala Glu Asp Tyr Pro Ile Asp
    130                 135                 140

Leu Tyr Tyr Leu Met Asp Leu Ser Tyr Ser Met Lys Asp Asp Leu Glu
145                 150                 155                 160

Asn Val Lys Ser Leu Gly Thr Asp Leu Met Asn Glu Met Arg Arg Ile
                165                 170                 175

Thr Ser Asp Phe Arg Ile Gly Phe Gly Ser Phe Val Glu Lys Thr Val
            180                 185                 190

Met Pro Tyr Ile Ser Thr Thr Pro Ala Lys Leu Arg Asn Pro Cys Thr
        195                 200                 205
Ser Glu Gln Asn Cys Thr Ser Pro Phe Ser Tyr Lys Asn Val Leu Ser
    210                 215                 220

Leu Thr Asp Lys Gly Glu Val Phe Asn Glu Leu Val Gly Lys Gln Arg
225                 230                 235                 240

Ile Ser Gly Asn Leu Asp Ser Pro Glu Gly Gly Phe Asp Ala Ile Met
                245                 250                 255

Gln Val Ala Val Cys Gly Leu Ile Met Gly Trp Arg Asn Val Thr Arg
            260                 265                 270

Leu Leu Val Phe Ser Thr Asp Ala Gly Phe His Phe Ala Gly Asp Gly
        275                 280                 285

Lys Leu Gly Gly Ile Val Leu Pro Asn Asp Gly His Cys His Leu Glu
    290                 295                 300

Asn Asp Val Tyr Thr Met Ser His Tyr Tyr Asp Tyr Pro Ser Ile Ala
305                 310                 315                 320

His Leu Val Gln Lys Leu Ser Glu Asn Asn Ile Gln Thr Ile Phe Ala
                325                 330                 335

Val Thr Glu Glu Phe Gln Pro Val Tyr Lys Glu Leu Lys Asn Leu Ile
            340                 345                 350

Pro Lys Ser Ala Val Gly Thr Leu Ser Ala Asn Ser Ser Asn Val Ile
        355                 360                 365

Gln Leu Ile Ile Asp Ala Tyr Asn Ser Leu Ser Ser Glu Val Ile Leu
    370                 375                 380

Glu Asn Ser Lys Leu Pro Glu Gly Val Thr Ile Asn Tyr Lys Ser Tyr
385                 390                 395                 400
Cys Lys Asn Gly Val Asn Gly Thr Gly Glu Asn Gly Arg Lys Cys Ser
                405                 410                 415

Asn Ile Ser Ile Gly Asp Glu Val Gln Phe Glu Ile Ser Ile Thr Ala
            420                 425                 430

Asn Lys Cys Pro Asn Lys Asn Ser Glu Thr Ile Lys Ile Lys Pro Leu
        435                 440                 445

Gly Phe Thr Glu Glu Val Glu Ile Ile Leu Gln Phe Ile Cys Glu Cys
    450                 455                 460

Glu Cys Gln Ser Glu Gly Ile Pro Ser Ser Pro Lys Cys His Asp Gly
465                 470                 475                 480

Asn Gly Thr Phe Glu Cys Gly Ala Cys Arg Cys Asn Glu Gly Arg Val
                485                 490                 495

Gly Arg His Cys Glu Cys Ser Thr Asp Glu Val Asn Ser Glu Asp Met
            500                 505                 510

Asp Ala Tyr Cys Arg Lys Glu Asn Ser Ser Glu Ile Cys Thr Asn Asn
        515                 520                 525

Gly Glu Cys Val Cys Gly Gln Cys Val Cys Arg Lys Arg Asp Asn Thr
    530                 535                 540

Asn Glu Ile Tyr Ser Gly Lys Phe Cys Glu Cys Asp Asn Phe Asn Cys
545                 550                 555                 560

Asp Arg Ser Asn Gly Leu Ile Cys Gly Gly Asn Gly Val Cys Lys Cys
                565                 570                 575

Arg Val Cys Glu Cys Asn Pro Asn Tyr Thr Gly Ser Ala Cys Asp Cys
            580                 585                 590
Ser Leu Asp Thr Thr Ser Cys Met Ala Val Asn Gly Gln Ile Cys Asn
        595                 600                 605

Gly Arg Gly Val Cys Glu Cys Gly Val Cys Lys Cys Thr Asp Pro Lys
    610                 615                 620

Phe Gln Gly Pro Thr Cys Glu Met Cys Gln Thr Cys Leu Gly Val Cys
625                 630                 635                 640

Ala Glu His Lys Glu Cys Val Gln Cys Arg Ala Phe Asn Lys Gly Glu
                645                 650                 655

Lys Lys Asp Thr Cys Ala Gln Glu Cys Ser His Phe Asn Ile Thr Lys
            660                 665                 670

Val Glu Asn Arg Asp Lys Leu Pro Gln Pro Gly Gln Val Asp Pro Leu
        675                 680                 685

Ser His Cys Lys Glu Lys Asp Val Asp Asp Cys Trp Phe Tyr Phe Thr
    690                 695                 700

Tyr Ser Val Asn Gly Asn Asn Glu Ala Thr Val His Val Val Glu Thr
705                 710                 715                 720

Pro Glu Cys Pro Thr Gly Pro Asp Ile Ile Pro Ile Val Ala Gly Val
                725                 730                 735

Val Ala Gly Ile Val Leu Ile Gly Leu Ala Leu Leu Leu Ile Trp Lys
            740                 745                 750

Leu Leu Met Ile Ile His Asp Arg Arg Glu Phe Ala Lys Phe Glu Lys
        755                 760                 765

Glu Lys Met Asn Ala Lys Trp Asp Thr Gly Glu Asn Pro Ile Tyr Lys
    770                 775                 780
Ser Ala Val Thr Thr Val Val Asn Pro Lys Tyr Glu Gly Lys
785                 790                 795

<210> 2
<211> 3831
<212> DNA
<213> Pig

<400> 2

GCGGCGGCGG CCTGAGGAGC TGGATTGCCG CGCGGAGGAG GCGGTGCCGC GGGGGTCGTT 60
TCCTGCCAGG AGGCGGCCCC GCGTCGCCGA GTCTCCTCCT CCCGCCCGCG AAGGAGGAGG 120
AGCTGCCGCC GCCGCGCCCG CGCCGCAGCC GGGGAGGCCA CGCCAGCCCT CGGACAGGCC 180
GAGCGGGAGT CGCGGAGCGC TGGTGGAGCC GACCGCGCGG GCCGGGCGCC GCGGGAGAAG 240
ATGAATTTAC AACTGATTTT CTGGATTGGA CTGATCAGTT CAGTTTGCTA TGTGTTTGGC 300
CAAGCAGATG AAAATAGATG TTTAAAAGCA AATGCCAAAT CATGTGGAGA GTGTATACAA 360
GCAGGGCCAA ATTGTGGATG GTGCACAAAT TCAACATTTT TACAAGAAGG AATGCCTACT 420
TCTGCTCGAT GTGATGATTT GGAAGCCTTA AGAAAGAAGG GTTGTCATCC AGATGACATT 480
GAAAATCCCA GAGGCTCCAA AAATATAAAG AAAAATAAAA ATGTAACCAA CCGTAGCAAA 540
GGGACAGCAG AGAAGCTGCA GCCGGAAGAT ATCACTCAGA TCCAACCACA GCAGTTGGTT 600
TTGCAGTTAC GATCAGGGGA GCCACAGACG TTTACGTTGA AATTCAAGCG AGCCGAGGAC 660
TATCCCATTG ATCTCTACTA CCTTATGGAC CTCTCCTACT CCATGAAAGA CGACTTGGAG 720
AACGTGAAAA GCCTTGGAAC AGATCTGATG AATGAAATGA GGAGGATTAC TTCAGACTTC 780
CGAATTGGAT TTGGCTCTTT TGTGGAAAAA ACTGTGATGC CTTACATTAG CACAACACCA 840
GCTAAGCTCA GGAACCCTTG CACAAGTGAA CAGAACTGTA CCAGCCCATT TAGCTACAAA 900
AATGTGCTCA GCCTTACTGA TAAAGGGGAA GTATTTAATG AACTTGTTGG TAAACAGCGC 960
ATATCTGGAA ATTTGGATTC TCCAGAAGGT GGCTTTGATG CAATCATGCA AGTTGCCGTT 1020
TGTGGATCTT TGATTGGCTG GAGGAATGTT ACACGGCTGC TGGTGTTTTC CACGGATGCC 1080
GGGTTTCACT TTGCCGGAGA TGGGAAACTT GGTGGCATTG TTTTACCAAA TGATGGACAT 1140
TGTCACCTGG AAAATGATGT ATACACAATG AGCCATTACT ACGATTATCC TTCTATTGCT 1200
CACCTTGTCC AAAAACTAAG CGAAAATAAC ATTCAGACGA TTTTTGCAGT TACTGAAGAA 1260
TTTCAGCCTG TTTACAAGGA ACTGAAAAAT TTGATCCCCA AGTCAGCAGT AGGAACATTA 1320
TCTGCAAATT CCAGCAATGT AATTCAGTTG ATCATTGATG CATACAATTC CCTTTCCTCA 1380
GAAGTCATTT TGGAAAACAG CAAATTACCA GAAGGAGTAA CAATAAATTA CAAATCTTAC 1440
TGCAAAAATG GGGTGAATGG AACAGGGGAA AACGGGAGAA AATGCTCCAA TATTTCCATT 1500
GGGGATGAGG TTCAGTTTGA AATTAGCATA ACTGCCAATA AATGTCCAAA TAAGAATTCT 1560
GAAACTATTA AAATTAAGCC TCTGGGCTTC ACTGAAGAAG TAGAGATAAT CCTTCAGTTC 1620
ATCTGCGAAT GTGAATGCCA GAGCGAGGGC ATCCCCAGCA GCCCCAAGTG CCATGACGGC 1680
AATGGGACGT TTGAGTGTGG GGCCTGCAGG TGCAACGAGG GACGTGTTGG GAGACATTGT 1740
GAATGTAGCA CAGACGAAGT GAACAGTGAA GACATGGACG CTTACTGCAG GAAAGAGAAC 1800
AGTTCAGAAA TCTGTACCAA CAATGGAGAG TGTGTCTGCG GACAGTGTGT ATGTAGGAAG 1860
AGGGATAATA CAAACGAAAT TTATTCTGGC AAATTCTGCG AGTGTGATAA TTTCAACTGT 1920
GATAGATCCA ATGGCTTAAT TTGTGGAGGA AATGGTGTTT GCAAGTGTCG AGTGTGTGAG 1980
TGCAACCCCA ACTACACTGG CAGCGCCTGT GACTGTTCTT TGGATACTAC GTCCTGCATG 2040
GCTGTAAATG GACAGATCTG CAATGGCCGG GGTGTCTGTG AATGTGGGGT CTGTAAGTGT 2100
ACAGATCCCA AGTTCCAAGG GCCAACTTGT GAGATGTGTC AGACCTGCCT CGGAGTCTGT 2160
GCTGAGCATA AAGAATGTGT TCAGTGCCGA GCCTTCAATA AAGGAGAAAA GAAAGATACA 2220
TGTGCCCAGG AATGTTCACA TTTCAACATT ACCAAGGTTG AAAATCGGGA CAAATTGCCC 2280
CAGCCAGGCC AGGTCGATCC CTTGTCCCAT TGTAAGGAGA AGGACGTTGA TGATTGCTGG 2340
TTCTACTTCA CATACTCGGT GAATGGGAAC AATGAGGCCA CTGTTCATGT TGTAGAGACT 2400
CCAGAGTGTC CCACTGGTCC AGACATTATT CCAATTGTAG CTGGTGTGGT TGCTGGAATT 2460
GTTCTCATTG GCCTTGCACT GCTGCTCATT TGGAAGCTTT TAATGATTAT TCACGACAGA 2520
AGGGAATTTG CCAAATTTGA AAAGGAAAAA ATGAATGCCA AATGGGACAC GGGTGAAAAT 2580

CCGATTTATA AGAGTGCCGT GACAACCGTT GTCAATCCGA AGTATGAGGG AAAATGAGCA 2640
GTGCTGCGCA GATCTCAGCA CTGAATGCCA AGTAGTAACT GCAGTCACAG TGAGGTAGCT 2700
TCAGGGCAGC GTCGCCAAGG GTCGCTATGG GCAGGTTCTG AAAATGTATA ATATGTATAA 2760
TTTGAAATTT TTTTTATTAT TTTGAAAATA ATGTTATCAC CAATGCCAGG GACTGACAAA 2820
AGACTTGAGA CGGGATGGTT ATCCTTGTCA GCTAAGGTCA CATTGTGCCT TTTTGACCTT 2880
TTCCTCGTGA ACTATTGAAG TGCAGCTCTC ATTGGATTAA GTGATATTTC TAGAGTGATT 2940
GAAAAGACAG TAGTTTAAAG TAATGAGTAT GGTGAGAGTA TCTGTTAGTC AGGTATTAAA 3000
ACTGATTTTT AGCTTTGCAG ATATGTCGGT TTTCAGTTAC ACAGAATCCA AAGTAAATGG 3060
TTTTCTAGCT AGTTTAGAAT TGTCTTAAGT TTATTATTTT GCTATTTGCC TAATATTAAG 3120
ACATGACTGA TGACATATCT GAGAGATGGG TATGTTGGGA GTTACTGGTG TAAAATAATG 3180
AAATATCTGA TCTCTAAAGG CCATAGGAAA AGTCAGTTGG GAGGGGAAAA ATGTAGCTTT 3240
AAAACCTGTG TGCCATTTTA AGAGTTACTT AATCCTGGTA ACTTTTATGC CTTCTCTTTT 3300
TAAATTCAAG CCTTGGATAA AAGTATTGGG AAATTTTCTG CAAAAAAAAG TTCTTTATTT 3360
AGCGTTATTT ACATAAAGGC CTTAATTTAC ATAGTACTTG CTAAATTCAG GACCTTTGGG 3420
TGAATTTATT TTTTTCATTT TTATTTTGTT TAAAGCCTGG TGCTTTTTAT CACCTCTTCT 3480
TCTAATCATT TAATGTATTT GTTTGCAATT TGGGGTAAGA CTTTTTTTTA ATCAGTACTT 3540
TTTTGTTGAT GTTTTAGCTG TACAGTTGCC TTTTAAATGA ACATGTGAAG TTGTACTGTG 3600
GCTTATGCAA CAGCTATCAC TTAAGCAGTT CTTACTTTCA TTTCAGTTAG TGCTGTAAGA 3660
GACCACTGTA TATTTACTCC TCACTGTTTG AATTCCGTTT TTTGTTTCAT ACTAGTCACA 3720
TTCTTATTTT AAGTGCCTTT TAATTTTAAC AGTTCACTTT TTACAGTGCT GTTTACTGAA 3780
GTTATTTATT GAATAAAAAT GCGTAAAATA TTTAAAAAAA AAAAAAAAAA A 3831

<210> 3
<211> 24
<212> DNA
<213> Artificial sequence

<220>
<223> Primer P1

<400> 3
CCACAGATGC CGGGTTTCAC TTTG 24

<210> 4
<211> 23
<212> DNA
<213> Artificial sequence

<220>

<223>	Primer P2

<400>	4
TGAATTGAAT GCCAAAGCGA AGG	23

## Description

### FIELD OF THE INVENTION

This invention is related to xenotransplants and, particularly, to an alternative to reduce or avoid hyperacute rejection associated with xenotransplants. The invention provides proteins, particularly recombinant proteins based on porcine protein CD29, nucleic acid molecules which code for said proteins and the use of said proteins and nucleic acid molecules in the development of strategies to reduce or avoid hyperacute rejection. Recombinant proteins based on porcine protein CD29 can have an amino acid sequence like that of wild-type CD29 or a modified amino acid sequence with an equal, reduced or zero expression of the epitope Gal-α-(1,3)-Gal.

### BACKGROUND OF THE INVENTION

Xenotransplantation consists in the transplantation of organs or tissues between members of different species. The clinical and scientific interest in this field has increased significantly due, among other causes, to the increased success of allogenic transplants (transplants of organs or tissues among non-genetically identical individuals which belong to the same species) and due to the fact that the supply of human organs is inadequate for the number of patients that may benefit from this type of therapy. All of this has motivated the scientific community to search for an alternative in xenotransplants. In order to obtain an idea of the dimensions of the problem suffice it to say that in the period comprised between 1990 and 1999 the number of transplants carried out annually in the world has increased by 30% whereas the number of candidates waiting for an organ has increased by over 100%. This increase would be larger, probably, if there were to be more available organs. However, currently the number of patients waiting for an organ is approximately 5 times the number of patients which receive one annually [H. Auchincloss Jr. And D. H. Sach, *Annu. Rev. Immunol*. 1998, 16:433-470, especially pages 434-435].

However, a problem associated with xenotransplants, for example, in the transplant of tissues or organs from a non-human species to the human species, limits their widespread use, this problem specifically being the hyperacute rejection which occurs due to the existence of natural antibodies present in the human serum which react against antigens present in the tissues of said non-human species, causing the rejection of the transplant within a short period of time. The binding of antigens reactive against a xenograft [graft of a tissue or an organ from one species into another] brings about the activation of complement which in turn mediates in the formation of lesions and injuries in the endothelial tissues of the blood vessels and in the loss of the functions of endothelial cells, which leads to the development of an interstitial haemorrhage and of a thrombosis which constitutes the typical symptoms of hyperacute rejection. A review of the problems associated with xenotransplants has been made by [H. Auchincloss Jr. and D. H. Sach, in "Xenogeneic Transplantation", published in *Annu. Rev. Immunol*. 1998, 16:433-470].

The importance of xenoreactive antibodies, i.e. antibodies from a species which react against antigens present in organs or tissues of other species, and the need to develop a source of alternative organs for their use in xenotransplantation therapies, has motivated the search for antigens of non-human species which are recognised by xenoreactive natural human antibodies. The identification of said antibodies would allow the development of specific ligands to remove the natural xenoreactive antibodies from the patients receiving a xenograft.

Xenograft rejection, particularly in the context of porcine tissue, is mostly associated with antibodies that react with the epitope identified as Gal-α-(1,3)-Gal [2 galactose residues bound by an α(1,3) bond], Holzknecht and Platt [*Journal of Immunology*, 1995, 154:4565-4575] have pointed out that glycoproteinaceous integrins α1, αv, α3, α5, β1 and β3 are, together with Willebrand's Factor and DM-GRASP, the main targets of natural human antibodies in porcine endothelial cells. However, they do not describe their molecular characterisation or the nucleotide sequence that encodes them, these being essential aspects for their cloning and use in the development of suitable strategies for their use in xenotransplant therapies.

Integrins are a family of glycoproteins which participate in the cell matrix and in cell-cell adhesion in many physiologically important processes, including embryological development, haemostasis, thrombosis, wound cicatrization, immune and non-immune defence mechanisms and oncogenic transformation. Integrins are composed by two non-covalently bound sub-units, α and β, which, associated in different combinations, generate functionally distinct heterodimeric complexes, with different binding specificities. The β1 subunit, also known as protein CD29, participates in six complexes with different functions. Complexes α1/β1 and α2/β1 are collagen receptors; complex α3/β1 has a wide specificity and binds to collagen, fibronectin and laminin; complex α4/β1 is a receptor that mediates in lymphocyte-target adhesion during cytolysis, as well as in the interaction of lymphocytes with endothelial cells; complexes α5/β1 and α6/β1 are specific receptors for fibronectin and laminin, respectively.

The cDNA sequences and the deduced amino acid sequences of CD29 proteins of man [Argraves, W., et al., *J. Cell Biol.*, 1987, 105:1183], mouse [Holers, V.M., at al., *J. Exp. Med.,* 1989, 169:1589], rat [Malek-Hedayat, S., et al., *Gene* 1995, 158:287], chicken [Tamkun, J.W., et al., *Cell,* 1986, 46:271] and *Xenopus laevis* [DeSimone D.W., et al., *J. Biol. Chem.,* 1988, 263:5333] have been published. The comparison of said sequences indicates that they are related, that they present a high degree of homology and that they share common structural characteristics such as identical transmembrane and cytoplasmatic domains and homologous cysteine-rich domains.

A factor that may contribute to the importance of integrins as targets of xenoreactive antibodies consists in that these structures are N-galactosylated in a relatively abundant manner. However, not all endothelial integrins are equally N-galactosylated. Among the different sub-units, sub-unit β1 [CD29] is the one that is most effectively recognised by human anti-Gal-α-(1,3)-Gal antibodies [Richard, C., et al., *Xenotransplantation*, 1998, 5:75], which could be the cause for which sub-unit β1 [CD29] is the preferred target of natural xenoreactive antibodies.

Although the most relevant epitope for the binding of human antibodies to porcine cells is Gal-α-(1,3)-Gal, there is some evidence that supports the idea that the expression of said epitope is not sufficient for the binding of complement-binding antibodies. In the first place, integrins present epitopes in three-dimensional dispositions that are specially favourable for the binding of xenoreactive antibodies. Secondly, natural xenoreactive antibodies make gaps in the endothelial monolayer that are similar to the effects produced by anti-CD29 antibodies. Both these evidences contribute to the idea of considering integrins, and particularly CD29, as mediators in the initial stages of hyperacute rejection.

Therefore there is the need to continue diving into the knowledge of the factors and proteins involved in hyperacute rejection in xenotransplant therapy, in order to develop strategies directed towards reducing or avoiding it.

### BRIEF DESCRIPTION OF THE INVENTION

It has now been possible to isolate and identify the cDNA that encodes porcine CD29, starting from a cDNA library prepared with messenger RNA from several porcine tissues, and its sequence, as well as the complete amino acid sequence, deduced from cDNA, have been determined. Additionally, the amino acid sequence of porcine CD29 has been compared with the amino acid sequences of other known CD29s, and the expression of CD29 has been studied in different porcine tissues.

The cloning and the characterisation of porcine CD29, the main target recognised by natural human xenoreactive antibodies, constitutes an important step in the development of specific ligands for the reduction or elimination of xenoreactive antibodies from xenograft recipients. Additionally, the determination of the sequence of porcine CD29 also means an important step in the understanding of the recognition of the Gal-α-(1,3)-Gal epitope by means of human natural xenoreactive antibodies and the possible reduction of hyperacute rejection by the genetic manipulation of the DNA molecule that codes for porcine CD29.

Therefore, an object of the present invention is constituted by a recombinant protein based on wild-type porcine CD29 or otherwise a modified amino acid sequence with an equal, reduced or zero expression of the Gal-α-(1,3)-Gal epitope. This recombinant protein may be used in several applications, for example, to remove natural xenoreactive antibodies, to induce the formation of antibodies against said protein or against a peptide fragment of the latter or to seek antagonists of the epitope-natural xenoreactive antibody binding. These applications, the methods based on these applications and the products resulting from the same constitute additional objects of the present invention.

An additional object of this invention is constituted by a nucleic acid molecule which encodes said porcine CD29 protein. Said nucleic acid molecule can have a nucleotide sequence such as the DNA sequence which codes for the wild-type CD29 protein, or otherwise, a nucleotide sequence modified in such a manner as to yield a modified porcine CD29 protein with an equal, reduced or zero expression of the Gal-α-(1,3)-Gal epitope. This nucleic acid molecule may be employed in several applications, for example to obtain a recombinant porcine CD29 protein, to construct vectors and cells that contain said DNA molecule, to obtain transgenic porcine cells, to obtain transgenic porcine organs and tissues suitable for xenotransplantation or that are resistant to rejection. These applications, the methods based on said applications and the products resulting from the same, constitute additional objects of the present invention.

### BRIEF DESCRIPTION OF THE DRAWINGS

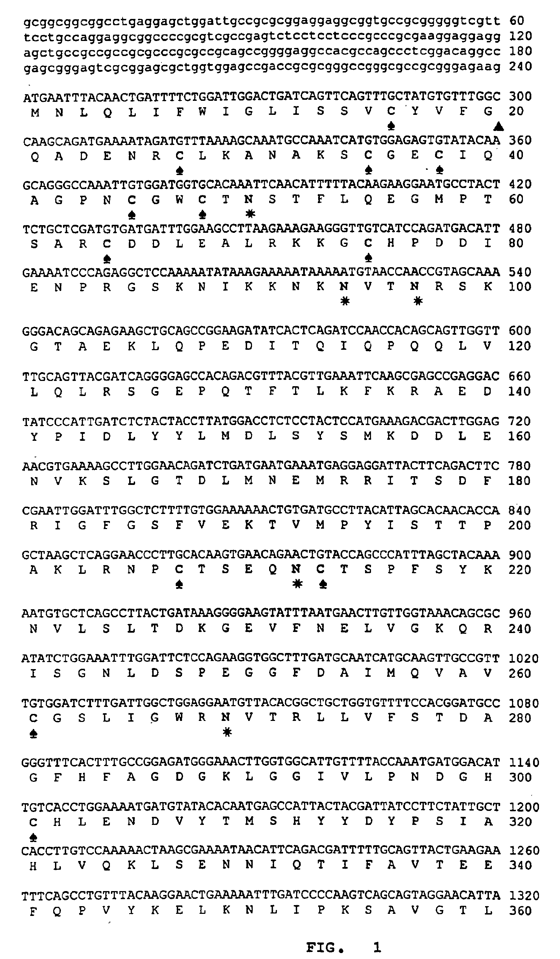

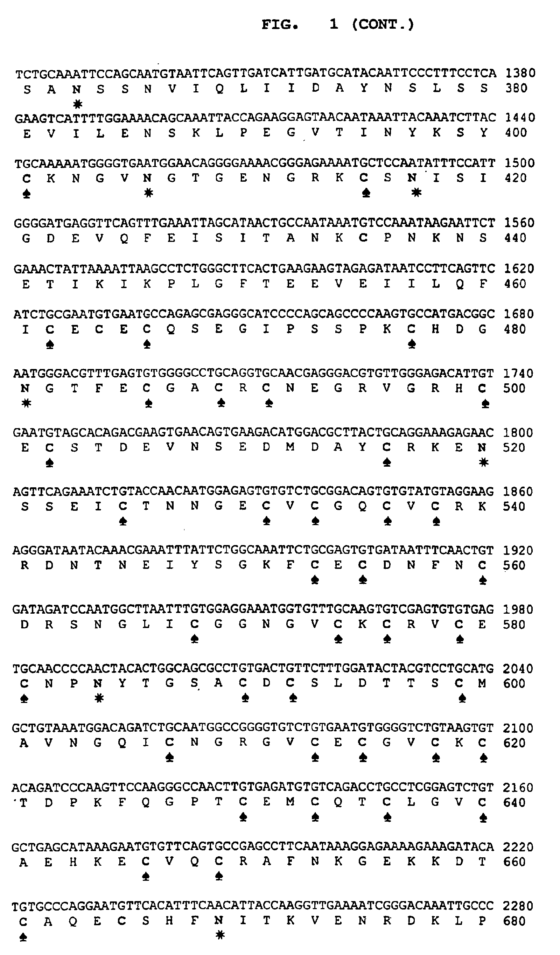

Figure 1 shows the cDNA sequence that codes for porcine CD29 and the complete amino acid sequence of porcine CD29 deduced from the cDNA. The putative excision site of the signal peptide has been indicated by a black triangle (▲). Potential sites for N-glycosylation have been marked with an asterisk (*) and the cysteine residue have been marked with a spade ( 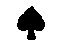 ). The putative transmembrane domain has been underlined.

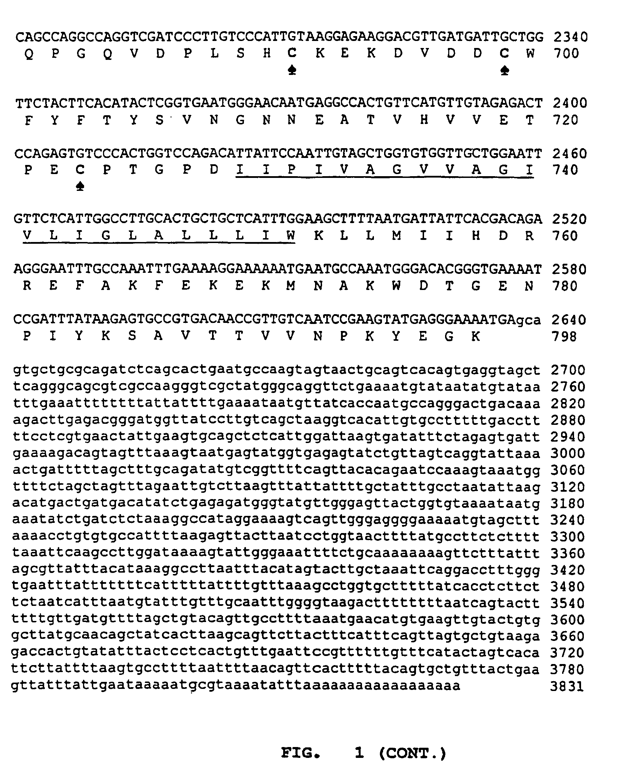

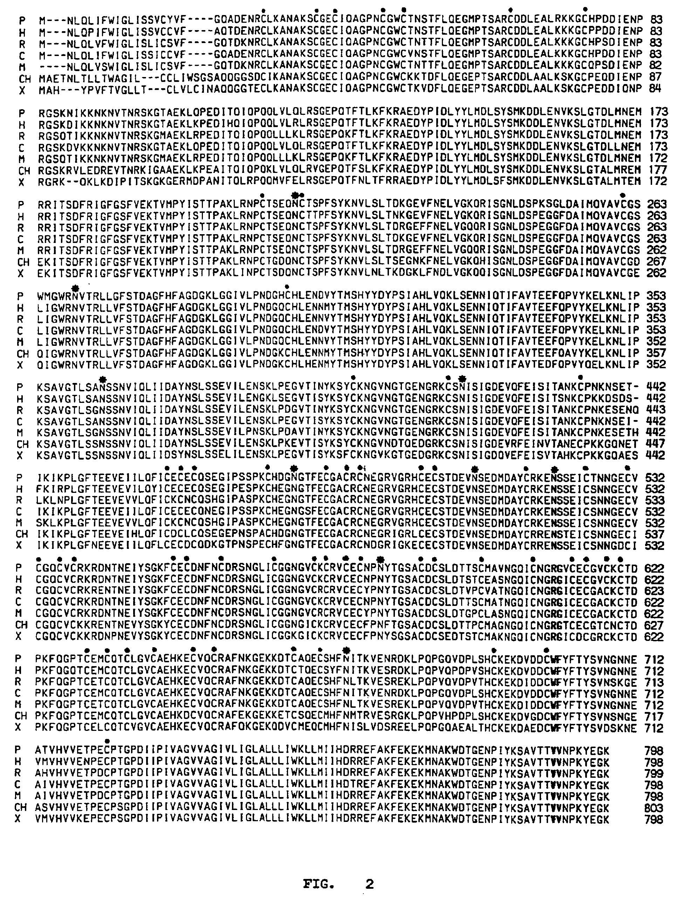

Figure 2 shows the comparison of the homology between the amino acid sequence of porcine CD29 and the amino acid sequences of the CD29 of 6 different species: human (H), rat (R), cat (C), mouse (M), chicken (CH) and *Xenopus laevis* (X). The dots (.) indicate the cysteine residues conserved in all the species. The N-glycosylation sites conserved in all the species are marked with an asterisk (*).

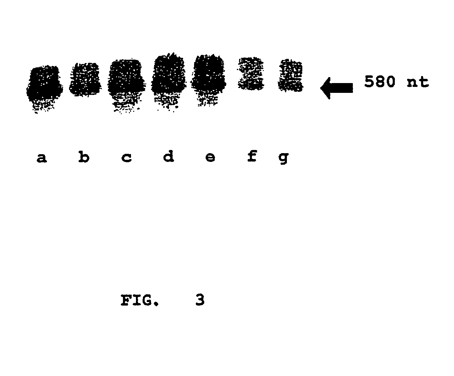

Figure 3 depicts the detection of mRNA corresponding to porcine CD29, by amplification, by means of the polymerase chain reaction (PCR), in different porcine tissues: a (spleen); b (thyme); c (alveolar macrophages); d (bone marrow); e (peripheral blood leukocytes); f (liver); and g (kidney).

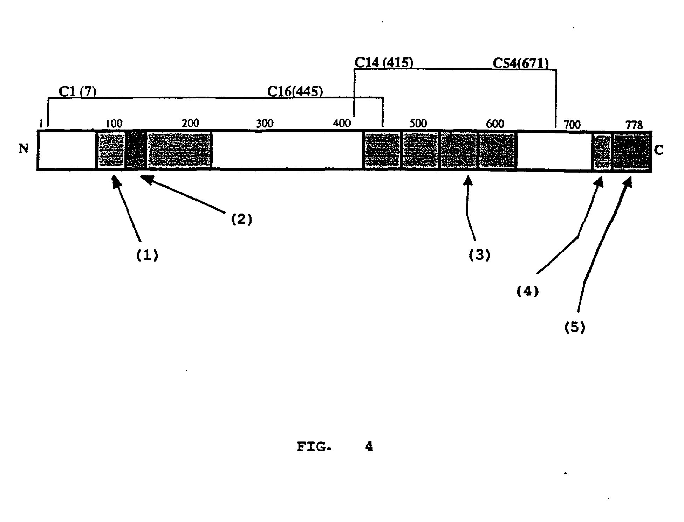

Figure 4 is a schematic representation of porcine CD29 that shows the different regions of this molecule: (1) ligand binding (67-230); (2) common region β (119-139); (3) 4 cysteine-rich regions (447-496) (497-539) (540-578) (579-626); (4) transmembrane region (719-741); and (5) cytoplasmic region (742-778).

### DETAILED DESCRIPTION OF THE INVENTION

The invention provides a recombinant porcine CD29 protein, from now on the porcine CD29 of the invention, that has an amino acid sequence selected from between:
a) The amino acid sequence shown in SEQ. ID. No.: 1, and
b) an amino acid sequence substantially homologous and functionally equivalent to the amino acid sequence shown in SEQ. ID. No.: 1.

In the sense used in this specification, the expression "substantially homologous" means that the compared amino acid sequences have a degree of identity, at the amino acid level of at least 70%, preferably of at least 85%, and even more preferably of at least 95%.

Likewise, in the sense used in this specification, the expression "functionally equivalent" means that the protein in question has at least one of the biological functions of porcine CD29, for example, in association with integrin α1 it acts as a receptor for porcine laminin and collagen, or otherwise, is recognised by monoclonal antibody NaM160-1A3 [Richard, C., et al., *Xenotransplantation*, 1998, 5:75].

Protein CD29 of the invention may have an amino acid sequence such as that of wild-type porcine CD29 or rather have a modified amino acid sequence with an equal, reduced or zero expression of the Gal-α-(1,3)-Gal epitope.

In the sense used in this description, the expression "protein with a modified amino acid sequence with an equal, reduced or zero expression of the Gal-α-(1,3)-Gal epitope" refers to a protein that has an amino acid sequence with one or several amino acids different from the wild-type porcine CD29 protein, the different amino acids changes including both those that affect the binding of the Gal-α-(1,3)-Gal epitope [sequence Asn-X-Ser-/Thr where X is not Pro] as well as changes that affect other domains, which include the ligand-binding domain, the first 220 amino acids of the amino-terminal end, and the regulatory domain, the last tree-hundred amino acids of the carboxyl-terminus. Likewise, it also includes the proteins that contain changes in the amino acid sequence that, although not affecting the expression of the Gal-α-(1,3)-Gal epitope, may have important effects upon the function of the molecule, for example, changes in the tertiary structure that make the epitopes inaccessible, without changes in the number of the latter.

In a particular embodiment, the CD29 of the invention has the amino acid sequence shown in SEQ. ID. No.:1 which corresponds to the putative amino acid sequence of wild-type porcine CD29.

Protein CD29 of the invention can be used in different applications, for example, to remove natural xenoreactive antibodies, to induce the formation of antibodies that recognise said protein or a fragment of the latter or to seek antagonists of the epitope-natural xenoreactive antibody binding.

The fragment of the CD29 protein of the invention is a polypeptide constituted by between 5 and 300 consecutive amino acids contained in any position of the amino acid sequence of protein CD29 of the invention. Said polypeptide, from now on referred to as the polypeptide of the invention, constitutes an additional object of the invention. In a preferred embodiment, the amino acid sequence of the polypeptide of the invention originates from the amino-terminal end of the protein CD29 of the invention, preferably, from the ligand binding domain, constituted by the first 220 amino acids of the amino-terminal end of protein CD29 of the invention, more preferably from the first 220 amino acids of the amino-terminal end of the amino acid sequence shown in SEQ. ID. No.: 1. In another preferred embodiment, the amino acid sequence of the polypeptide of the invention originates from the carboxyl-terminal end of the protein CD29 of the invention, preferably, from the regulatory domain, constituted by the last 300 amino acids of the carboxyl-terminal, more preferably from the last 300 amino acids of the carboxyl-terminal of the amino acid sequence shown in SEQ. ID. No.: 1.

The polypeptide of the invention may be used, among other applications, to induce the formation of antibodies that specifically recognise said polypeptide to study, for example, the binding of the protein to the ligand, and its regulation.

Antibodies (polyclonal or monoclonal) can be obtained that recognise the protein CD29 of the invention, or a polypeptide of the invention, which constitute an additional object of this invention, by means of conventional techniques, for instance, by inoculation of said protein or polypeptide into an animal, followed by the extraction of the antibodies. Alternatively, the cells that produce said antibodies can be fused to an immortal cell line to form a hybridoma capable of producing monoclonal antibodies that specifically recognise protein CD29 of the invention, or the polypeptide of the invention.

Protein CD29 of the invention can also be used as a model for the screening of compounds which are antagonists of the epitope-natural xenoreactive antibody binding, that is, compounds capable of blocking, hindering or reducing the binding of natural xenoreactive antibodies with the different epitopes eventually present in porcine CD29 protein, among them the Gal-α-(1,3)-Gal epitope. Said antagonistic compounds could be potential candidates for their use in xenotransplant therapy to avoid the rejection of the xenograft, particularly, hyperacute rejection.

The CD29 of the invention can also be used to remove human natural xenoreactive antibodies which recognise porcine CD29 by means of appropriate techniques, for example, by means of affinity chromatography, fixing the CD29 of the invention to a column and passing through it the blood or the serum of the receptor of the xenograft, before the xenotransplant is carried out, with the object of reducing or eliminating the rejection of the xenograft. Alternatively, the CD29 of the invention can be administered directly to the receptor of the xenograft, with the object of eliminating or reducing the human natural xenoreactive antibodies formed.

Therefore, the invention provides a method to remove human natural xenoreactive antibodies that recognise porcine protein CD29, which comprises putting into contact a CD29 protein of the invention with said antibodies, under conditions that permit the formation of an antigen-antibody complex. In a particular and preferred embodiment, said method is carried out in vitro fixing the CD29 protein of the invention to a column suitable for affinity chromatography and passing over said column the blood or serum of an individual under conditions that allow the formation of an antigen-antibody complex. In another particular embodiment, said method is carried out in vivo and comprises administering protein CD29 of the invention, or a pharmaceutical composition containing it, to the receptor of the xenograft. In a particular embodiment, protein CD29 of the invention is presented in a form suitable for its parenteral administration, preferably intravenously. Consequently, the necessary pharmaceutically acceptable excipients are added for said form of administration. Once the CD29 of the invention is introduced into the bloodstream of the receptor of the xenotransplant, the CD29 of the invention attracts the human natural xenoreactive antibodies, reacting with them and forming an antibody-antigen type complex.

The invention also provides a method to reduce or eliminate hyperacute rejection in xenotransplants due to the reaction between porcine CD29 protein and human natural xenoreactive antibodies that recognise said CD29 porcine protein, which comprises, prior to introducing the xenograft into the patient that is going to receive the porcine xenograft, putting the blood or the serum of the patient, which contains said human natural xenoreactive antibodies, in contact with a CD29 porcine protein of the invention, under conditions which allow the formation of an antigen-antibody-complex, and removing said antigen-antibody complexes from the blood or the serum of the patient. In a particular embodiment of this method, *in vitro,* the formation of the antigen-antibody complex and the removal of the antigen-antibody complexes from the blood or serum of the patient are carried out by means of affinity chromatography, attaching a CD29 protein of the invention to a suitable column and passing over said column the blood or the serum of the patient which is going to receive the xenograft. In another particular embodiment of this method, *in vivo*, protein CD29 of the invention, or a pharmaceutical composition containing it, is administered to the recipient of the xenograft. In a particular embodiment, protein CD29 of the invention is presented in a suitable form for its parenteral administration, preferably by the intravenous route.

Protein CD29 of the invention can be obtained from cells that produce it by means of a process comprising the culture of said cells under appropriate conditions for the expression of said protein and the recovery of the latter. Therefore, the invention also provides a method for the production of CD29 of the invention, which comprises culturing a suitable host cell which contains a DNA molecule which codes for said protein, or a DNA construct that contains it, under conditions which permit the production of said protein and its harvesting from the culture medium.

Likewise, the polypeptide of the invention can be obtained by culturing a suitable host cell which contains a DNA molecule of nucleic acid which codes for said polypeptide, or a DNA construct that contains said DNA molecule, under conditions which permit the production of said polypeptide and its harvesting from the culture medium. The nucleic acid molecule that encodes the polypeptide of the invention, as well as the DNA construct containing said nucleic acid molecule, constitute additional objects of the present invention. Alternatively, the polypeptide of the invention, depending on its size, can be obtained by chemical peptide synthesis techniques well known in the art, for example using Fmoc chemistry [Fields, C.G., et al., *Peptide Res.,* 1991, 4::95-101].

The invention also provides a nucleic acid molecule that codes for the CD29 of the invention, nucleic acid molecule of the invention, which comprises:
a) a DNA sequence identified as SEQ. ID. No.: 2; or
b) a DNA sequence analogous to the DNA sequence defined in a) which
   i) is substantially homologous to the DNA sequence defined in a); and/or
   ii) encodes a polypeptide that is substantially homologous to the protein encoded by the DNA sequence defined in a).

In the sense used in this description, the term "analogous" pretends to include any DNA sequence that codes for a porcine CD29 that has the properties i) and/or ii) indicated above. The analogous DNA sequence can be isolated from another species that produces a CD29 on the basis of the DNA sequence shown in SEQ. ID. No.: 2, or can otherwise be constructed based on the DNA sequence shown in SEQ. ID. No.: 2, for example, by the introduction of conservative or non-conservative nucleotide substitutions. Other examples of possible modifications include the insertion of one or more nucleotides, the addition of one or more nucleotides in any of the extremes of the sequence, or the deletion of one or more nucleotides in any extreme or in the interior of the sequence.

In the sense used in this specification, the expression "substantially homologous" applied to nucleotide sequences, means that the compared nucleotide sequences have a degree of identity, at the nucleotide level, of at least 70%, preferably of at least 85%, and even more preferably of at least 95%.

The nucleic acid molecule of the invention can be constituted by genomic DNA (gDNA) or by copy DNA (cDNA)and can have a nucleotide sequence such as the DNA sequence that codes for wild-type CD29 protein, or rather a nucleotide sequence modified so that it yields a modified porcine CD29 protein with an equal, reduced or zero expression of the Gal-α-(1,3)-Gal epitope. In a particular embodiment, the DNA molecule of the invention is a cDNA molecule that codes for a porcine CD29 that has the nucleotide sequence shown in SEQ.ID.No.:2.

The nucleic acid molecule of the invention can be obtained by the use of conventional techniques know in the art, from animals of the porcine species or from a host organism transformed with said nucleic acid molecule, for example, transformed *Escherichia coli* bacteria.

In a particular embodiment, the nucleic acid of the invention can be obtained by means of a process comprising the creation of gDNA or cDNA libraries starting from the RNA of cells and tissue that produce porcine CD29, the design of specific probes against porcine CD29, the screening of said libraries and the selection of positive clones. Example 1 describes the isolation of cDNA clones that encode porcine CD29 from a cDNA library prepared from the RNA of different porcine tissues, and the screening of said library with probes specific for porcine CD29, obtained from oligonucleotides based on the cDNA sequence that codes for human CD29.

The nucleic acid molecule of the invention can be used in diverse applications, for example, to obtain the protein CD29 of the invention by the use of suitable Genetic Engineering techniques, as well as to construct vectors and cells that contain said nucleic acid molecule, to obtain (i) transgenic porcine cells, (ii) transgenic porcine tissues and organs suitable for xenotransplantation, or (iii) transgenic pigs the organs and tissues of which are resistant to rejection.

The invention also provides a DNA construct that comprises the nucleic acid molecule of the invention and a transcription initiator region functional in the chosen expression system. Said DNA construct may also contain an operatively linked transcription termination sequence.

The nucleic acid molecule of the invention, or the DNA construct containing it, can be inserted into a suitable vector. Therefore, the invention also provides a vector, such as an expression vector, which comprises said nucleic acid molecule of the invention or said construct containing it. The choice of the vector will depend on the host cell into which it is going to be subsequently introduced. By way of example, the vector into which said molecule or DNA construct is introduced can be a plasmid or a vector that, when introduced into a host cell, becomes integrated within the genome of cells and replicates together with the chromosome (or chromosomes) into which it/they have integrated. As an example of a host cell it is possible to mention the CHO cell line (Chinese hamster ovary) and mutants developed from it, such as DG44.

In the vector provided by this invention, the nucleic acid molecule of the invention has to be operatively attached to a promoter and to a terminator sequence. The promoter may be any DNA sequence showing a transcriptional activity in the chosen host cell, and may derive from genes that code for either homologous or heterologous proteins of the host cell. The procedures used to ligate the nucleic acid sequence of the invention to the promoter and to the terminator sequence, respectively, and to insert said DNA construct into a vector are well known in the art and have been described, for example, by Sambrook at al.[Molecular Cloning. A Laboratory Manual, Cold Spring Harbor, NY, 1989].

The invention also provides a cell comprising the nucleic acid molecule of the invention, or a DNA construct containing it, or the vector mentioned above. The host cells that may be transformed with the DNA sequence of the invention can be, preferably, eukaryotic, capable of glycosylating the protein expressed by the nucleic acid molecule of the invention, or by the DNA construct that contains it, such as cells from animal tissues, for example, the CHO cell line and mutants developed from it such as DG44, insect cells or cells from plant tissues. The transformation of these cells can be carried out by conventional means, collected for example by Sambrook et al. [cited *supra*]. In a particular embodiment, the transformed cells are cells from porcine tissues.

The nucleic acid molecule of the invention, particularly when it contains a nucleotide sequence modified in relation with the DNA sequence which codes for wild-type porcine CD29 protein, so that it yields a modified porcine CD29 with an equal, reduced or zero expression of the Gal-α-(1,3)-Gal epitope, can be suitable for obtaining transgenic animals, particularly pigs, with transgenic biological material (cells, organs and tissues) in which protein CD29 is a modified protein which shows a reduced or zero expression of the Gal-α-(1,3)-Gal epitope or in which said epitope(s) are (were) inaccessible due to changes in the tertiary structure of the protein. Said transgenic biological material could be used as a xenograft in xenotransplant therapy as they would be less susceptible of rejection in humans and, consequently, would contribute to avoid or reduce the rejection of the xenograft, particularly the hyperacute rejection associated with xenotransplants.

Transgenic animals can be obtained by means of conventional techniques known by those skilled in the art, for example, by means of the introduction of the desired genetic material into germinal cells of the animal, or by means of the introduction of said genetic material in embryonary cells which are subsequently introduced into the embryo, or by altering cells originating from the animal to be modified, for example, bone marrow cells, and reintroducing them, once modified *in vitro.* The genetic material to be introduced can be, for example, a nucleic acid molecule of the invention that has a nucleotide sequence modified in relation to the DNA sequence which codes for wild-type porcine CD29 protein, so that it yields a modified porcine CD29 with an equal, reduced or zero expression of the Gal-α-(1,3)-Gal epitope, or a DNA construct that contains the nucleic acid molecule of the invention. Said genetic material can be introduced into the genome of the animal to be transformed, by several techniques, for example, by microinjection of the DNA into fertilised eggs, by retroviral infection of embryos, or otherwise through pluripotent embryonary cells, isolated from the internal cell mass of blastocysts [ES cells (embryo derived stem)] in which the DNA has been introduced by transfection or by retroviral infection. A review on gene transfer to whole organisms and the obtention of transgenic animals can be found, for example, in the text book entitled "Ingenieria Genética", authored by Marta Izquierdo, edited by Ediciones Pirámide, S.A., Madrid, 1993, Chapter 8, pages 175-197. Transgenic animals which have transgenic biological material, for example transgenic cells, organs and tissues which contain a DNA molecule of the invention, as well as said transgenic biological material and its use as xenografts in xenotransplant therapy, constitute additional objects of the present invention.

The following examples serve to illustrate the present invention and must not be considered as limiting of the scope of the same.

### EXAMPLE 1

### Determination of the cDNA sequence that codes for porcine CD29

### 1.1. RNA Isolation

Tissues were taken from adult pigs immediately after sacrifice. Peripheral blood lymphocytes (PBL) were obtained from complete heparinised blood by means of a density gradient in Ficoll-Hypaque. Total RNA was purified by the "Tripure Isolation Reagent" (Boehringer Mannheim) method and was quantified by absorption spectrometry at A₂₆₀/A₂₈₀. The RNA samples were maintained at -20°C after controlling its quality on a denaturing agarose gel.

### 1.2. Synthesis of specific probes for porcine CD29

cDNA probes were synthesised by RT-PCR using total RNA from a pig's spleen. To do this, 10 µg of RNA, resuspended in 9.5 µl of water, were heated for 3 minutes at 65 °C in the presence of random hexamers (final concentration 7.5 µM; Pharmacia) and, subsequently, were cooled in ice. The RNA was subjected to reverse transcription using 200 units of Moloney Leukaemia Virus reverse transcriptase (Gibco BRL) for 1 hour at 42 °C in a final volume of 20 µl that contained 4 µl of 5X reverse transcriptase buffer, 25 units of ribonuclease inhibitor (Boehringer Mannheim), 1 µl of each deoxynucleoside triphosphate (dNTP) 20 mM and 2 µl of 0.1 M dithiothreitol. At the end of the reaction, the solution was heated for 10 minutes at 95°C and was cooled in ice. 2 µl of this mixture were subjected to 35 PCR cycles using *Tth* DNA polymerase [Biotools, Biotechnological & Medical Laboratories, S.A., Madrid] and two primer oligonucleotides, direct (P1) [SEQ.ID.No.: 3] and reverse (P2) [SEQ. ID. No.: 4], based on the cDNA sequence that codes for human CD29 [Argraves, W., et al., *J. Cell Biol.,* 1987, 105:1183]. Each cycle consisted of incubations at 94°C for 1 minute, 55°C for 1 minute and 72°C for 1 minute. Finally, the elongation was extended for 10 minutes at 72°C. The 580 base pair (bp) cDNA fragment amplified was subcloned in the pGEM-T vector (Promega) and was sequenced as described further below.

### 1.3. Labelling of the CD29-specific probes

The CD29-specific probes were labelled using the PCR kit "DIG Probe Synthesis" (Boehringer Mannheim) following the manufacturer's instructions.

### 1.4. Isolation of the cDNA clones

A cDNA library prepared from several tissues [Nunes, M., et al., *Mamm. Genome,* 1994, 5:616] with the ZAP-cDNA Synthesis kit (Stratagene) was screened, under stringency conditions, using the probe P1P2-CD29 specific for CD29 [prepared using the primer oligonucleotides P1 and P2 (see section 1.2.)] marked with DIG (Digoxigenin). The method followed to detect positive plaques in transferred nylon membranes (Nytran N, Schleicher & Schuell) was the Genius System (Boehringer Mannheim). The membranes were incubated overnight at 42°C in a hybridisation solution [50% formamide, 5X SSC, 0.1% Sarcosyl, 0.02% SDS (sodium dodecyl sulphate), 1% blocking agent and 10 ng/ml of DIG-labelled P1P2-CD29 probe]. After washing the membranes in 0.5X SSC (saline sodium citrate), 0.1% SDS, twice, for 15 minutes at 65°C, detection was carried out using a colorimetric method with substrate BCIP/NBT (5-Bromo 4-Chloro 3-Indolyl phosphate 4-toluidine salt - Nitrobluetetrazolium) using a "DIG Detection Kit" (Boehringer Mannheim). The inserts from the purified plaques were recovered as a pBluescript phagemid by *in vivo* excision of the parental phage lambda as described by Stratagene.

### 1.5. Sequencing of the cDNA that encodes porcine CD29

The sequencing of the cDNA was carried out using an "ABI PRISM Terminator Cycle Sequencing Kit" (PE Applied Biosystems). The sequencing reactions were carried out in a GeneAmp PCR System 2400 DNA thermocycler (Perkin Elmer) following the manufacturer's instructions and were analysed in an ABI PRISM 310 sequencer (PE Applied Biosystems).

### 1.6. Results

Screening under stringency conditions with the P1P2-CD29 probe (specific for porcine CD29) of a cDNA library constructing using mRNA of different porcine tissues allowed the identification of several positive clones. The complete cDNA sequence of porcine CD29 was obtained from a single clone identified as cd29-4.2. The analysis of the sequence of the insert revealed a cDNA sequence of 3,831 bp which contained a 240 bp untranslated 5' flanking region, a single open reading frame that encoded a 798 amino acid polypeptide and a 1,194 bp untranslated 3' flanking region [see Figure 1].

The amino acid sequence deduced for mature porcine CD29 presents the typical structure, with small differences, of the β1 chains of integrins, and is summarised schematically in Figure 4.

In the amino region of porcine CD29, the first twenty amino acids are predominantly hydrophobic and correspond to the putative sequence of the signal peptide, with an identity of 17 out of 20 amino acids with respect to human CD29. There are 23 hydrophobic amino acids in the terminal carboxyl end which probably constitute the transmembrane domain [see Figure 1, underlined]. There is a sequence of 47 amino acids next to the putative transmembrane domain that probably represent the cytoplasmic domain of the molecule.

Excluding the signal sequence, the sequence of porcine CD29 consists of 778 amino acids with an estimated molecular weight of 88,277 daltons. The sequence of porcine CD29 contains 12 potential N-glycosylation sites [Asn-X-Ser-/Thr where X is not Pro]. Assuming that the hydrocarbon chains, with an average molecular weight of 2,500 dalton, were to bind to the 12 putative glycosylation sites, the total molecular weight of the mature porcine CD29 molecule would be of approximately 116,248 daltons. This value is consistent with the size for porcine CD29 obtained by SDS-PAGE [Richard, C., et al., *Xenotransplantation*, 1998, 5:75].

The deduced amino acid sequence of the extracellular domain of porcine CD29 has 708 amino acids. Except for a cysteine that is situated in the signal peptide, the other 56 cysteine residues are in the extracellular domain. Many of the cysteines (31) are arranged in a cysteine-rich domain with a sequence that is homologously 4 time within a fragment of 180 amino acids found near the transmembrane domain (446 to 626 residues).

The two partial amino acid sequences previously published for porcine CD29 [Holznecht, Z.E., and Platt, J.L., Journal of Immunology, 1995, 154:4565-4575; and Richard, C., Xenotransplantation, 1998, 5:75] correspond to internal peptides (NNIQTIFAXT and KWDTGENPIY, respectively) which have now been proved to correspond to residues 299 to 308 and 774 to 783 in the extracellular and cytoplasmatic domains, respectively. In the case of the peptide of the extracellular domain, the inventors have deduced that, as in humans, X corresponds to a V.

### EXAMPLE 2

### Expression of porcine CD29

In order to study the expression of porcine CD29 mRNA in different tissues, an RT-PCR was carried out following a process similar to that described in Example 1.2. To do this, 10µg of total RNA from lymphoid tissues (spleen, thyme, PBLs, bone marrow, alveolar macrophages) and non-lymphoid tissues (kidney and liver) were used, as well as primer oligonucleotides P1 [SEQ.ID.No.: 3] and P2 [SEQ.ID.No.: 4] to generate a band of porcine cDNA of 580 bp. The amplification products were subjected to electrophoresis in a 1X TAE gel with 1% agarose, the DNA was transferred to nylon membranes using a VacuGene XL (Pharmacia LKB) and were fixed by heating at 80°C for 2 hours. Hybridisation of the membrane with DIG labelled P1P2-CD29 probe and detection were carried out according to the methodology described in relation to the isolation of cDNA clones (Example 1.4.).

The obtained results showed that porcine CD29 has a high level of expression in lymphoid tissues and a lower level of expression in non-lymphoid tissues (Figure 3).

The expression model of porcine CD29 mRNA indicates that, as in the case of its human homologue, it can be expressed ubiquitously. However, in contrast with the expression of CD29 in humans, the findings indicate that the level of expression in lymphoid tissues is greater than in non-lymphoid tissues.

### EXAMPLE 3

### Comparison of CD29 proteins of different species

The homology between all known cloned CD29 proteins of different species (Figure 2) shows a very high conservation degree 97,5% amino acid identity with feline CD29 [Willet, 1995, GeneBank Accession: U27351], 94.4% with human CD29 [Argraves, W., et al., *J. Cell Biol.,* 1987, 105:1183], 92.7% with murine CD29 [Holers, V.M., at al., *J. Exp. Med.*, 1989, 169:1589], 92.5% with CD29 of rat [Malek-Hedayat, S., et al., *Gene* 1995, 158:287], 84.8% with CD29 of chicken [Tamkun, J.W., et al., *Cell,* 1986, 46:271] and 80.5% with CD29 *of Xenopus laevis* [DeSimone D.W., et al., *J. Biol. Chem.,* 1988, 263:5333]. In all these proteins there is an absolute conservation of 56 cysteine residues throughout the whole protein. All the CD29 sequences contain cysteine-rich homologous repeats and display total identity in the different cytoplasmatic and transmembrane domains. Finally, 12 potential glycosylation sites are conserved in mammals, but not in chicken or *Xenopus*, the more evolutionary distinct species.

The porcine CD29 molecule has been compared with other 6 previously described CD29s (man, cat, rat, mouse, chicken and *Xenopus laevis*). The human, feline and porcine CD29 proteins have the same number of amino acids in the extracellular domain (708), in the signal peptide (20), in the transmembrane domain (23) and in the cytoplasmic domain (47). All these CD29 described share common structural elements including the cysteine-rich homologous repeat domains and a similar number of potential glycosylation sites, 12, in all the studied mammals. Porcine CD29 presents 100% identity in the residues of the transmembrane and cytoplasmic domains with the other known CD29s, including *Xenopus laevis* and chicken, which seems to indicate that the possible mechanism for cellular activation (phosphorylation of some cellular proteins) through subunit β1 of integrin is conserved, at least within the species studied. The cysteine residues that are homologous with the cross-linked ones in β3, to form two long-range disulphide bridges (19) are connected by lines in Figure 4. These include the first cysteine (C1) which occupies position 7 paired with the sixteenth cysteine (C16) which occupies position 444, and the fourteenth cysteine (C14) in position 415 paired with the fifty-fourth cysteine (C54) in position 671.

### EXAMPLE 4

### Putative applications of CD29 of the invention

Two important domains have been defined in human and chicken CD29, the ligand-binding domain and the regulatory domain, by means of studies of epitopes for monoclonal antibodies [Shih, D., et al., *The J. Cell Biol.,* 1993, 122:1361; Takada, Y. and Puzon, W., *J. Biol. Chem.*, 1993, 268:17597]. The sequences of human and porcine CD29 have been compared and the differences between them in relation to the ligand-binding domain and the regulatory domain have been determined. It has been found that there are 9 point changes in the ligand-binding domain and 25 in the regulatory domain. The point changes in these porcine domains may have as a result differences in the ability to recognise human fibronectin, laminin, collagen and vitronectin, with important consequences for cellular adhesion in processes involving the transplant of porcine organs.

The study of the epitopes of human and chicken CD29 with monoclonal antibodies [Shih, and Takada and Puzon, cited *supra],* concluded that the 220 residues of the amino terminal of the β1 subunit of integrin are responsible of ligand-binding; previously Takada et al. [Takada, Y., at al., J. Cell. Biol., 1992, 119:913] had demonstrated that a point mutation in amino acid 130 of human CD29, was capable of stopping fibronectin binding. Comparing these 220 terminal amino acids between the sequences of human and porcine CD29, the inventors have found 9 point changes, one of them being the replacement of Asn²⁰⁷ with Asp, named N207D. N207D is situated in the TDKG sequence (residues 206-209), where a β-helix is predicted in the human CD29 sequence [Takada, Y. and Puzon, W., cited *supra*]. The binding of antibodies to the structure of the β-helix produces the activation or the inhibition of ligand binding. A flow cytometry analysis, with CHO cells that expressed a human β1 subunit mutated at residue 207 (N207D), of nine monoclonal antibodies specific against human CD29, disclosed that the binding of monoclonal antibodies was reduced but was not eliminated by this point mutation. Assuming that a point mutation is capable of blocking or reducing ligand binding, any of the changes described here between porcine CD29 and human CD29 could imply an inability of porcine endothelial cells to recognise human fibronectin, laminin, collagen and vitronectin molecules.

The other functional part important in CD29 is the regulatory domain, which includes the domain for the interaction of the α subunit [Shih, D., et al., cited *supra].* The regulatory domain is located within the approximately 300 amino acids of the carboxyl terminal end which are closest to the transmembrane domain. The binding of monoclonal antibodies to the regulatory domain results in the loss or in the increase of the ability of integrin to recognise and/or to be able to bind another [Shih, D., and Takada, Y. and Puzon, W., cited *supra].* A monoclonal antibody (QE.2E5) which recognises an epitope between residues 427 and 597 of human CD29 is capable of inducing a high affinity binding between fibronectin and integrin α5β1 [Faull, R.J., et al., *J. Biol. Chem.,* 1996, 271:25099]. The regulatory domain of CD29 includes the domains of the cysteine-rich repetitions, which comprise the residues between amino acids 446 and 626. Comparing the sequences of the regulatory domains of human and porcine CD29, the inventors have found 25 changes. The changes found in the domains of the cysteine-rich repetitions would have a special relevance. The most important groups of changes with respect to human CD29 are located near the ends of the domains of the cysteine-rich repetitions; 7 changes situated between residues 412 and 423 (42% homology) and 4 changes concentrated between residues 577 and 582, with only 30% homology. The differences found within the regulatory domain and in the domains of the cysteine-rich repetitions of porcine CD29 with respect to human CD29 could also affect, such as point changes in the ligand binding domain, the binding ability or affinity of porcine tissues to human fibronectin, laminin, collagen and vitronectin molecules.

A study using monoclonal antibodies against the ligand-binding domains and/or the regulatory domains will permit to elucidate which are the most critical residues for ligand binding and to assess the possibility of blocking natural human antibodies against the Gal-α-(1,3)-Gal epitope, as well as the development of new protocols for reducing or avoiding the hyperacute rejection associated with xenotransplants, which would comprise the administration of protein CD29 of the invention to the receptor of the xenograft, in substitution of Gal-α-(1,3)-Gal molecules.

Porcine CD29, as part of integrins α3β1 and α5β1, is one of the main targets of human natural xenoreactive antibodies. Most human natural antibodies recognise the Gal-α-(1,3)-Gal epitope bound to N which are expressed in the integrin molecules of endothelial cells. The avidity of human natural antibodies for the Gal-α-(1,3)-Gal epitope on purified porcine integrins is greater by 7 orders of magnitude to that of said antibodies for isolated Gal-α-(1,3)-Gal [Holzknecht, Z.E., and Platt, J.L., *Journal of Immunology,* 1995, 154:4565-4575]. The idea that natural antibodies can preferentially employ certain antigens has been analysed in detail by Holzknecht and Platt [cited *supra*], who postulated that the binding of natural antibodies to the surface of porcine cells, and the activation of complement may be facilitated by factors other than the expression of a target epitope [Gal-α-(1,3)-Gal]. Among these factors one could count the visualisation of epitopes in three-dimensional arrangements over glycoproteins and the post-transcriptional modifications common to the integrin family. According to the hypothesis of Holzknecht and Platt, the cloning of the gene that encodes the porcine CD29 protein could provide a more efficient protocol for the process of removing human natural xenoreactive antibodies before transplant than the methods employed to date to prevent hyperacute rejection of xenografts [Greenstein, J.L., and Sachs, D., The use of tolerance for xenotransplantation across xenogeneic barriers. *Nature Biotechnology.* Vol. 15 March 1997, pages 235-238, particularly, page 236]. After the cloning of the gene that encodes porcine CD29 it would be possible to use new column based protocols that carry porcine CD29 instead of the current isolated Gal-α-(1,3)-Gal molecules bound to silica.

The possibility that the protein of the invention may be used to remove human natural xenoreactive antibodies can be deduced from the work of Hoizknecht and Platt (cited *supra)* in Figures 3 and 4 of which, results are presented of the reactivity of human natural antibodies with extracts of porcine endothelial cells, being it possible to observe that one of the molecules recognised is β1.

The cloning of CD29 could also provide a new perspective to the pathogenesis of xenotransplant rejection, opening the possibility of removing or reducing the expression of the Gal-α-(1,3)-Gal determinant trough the genetic manipulation of the gene that codes for porcine CD29.

### DEPOSIT OF BIOLOGICAL MATERIAL

The culture of *Escherichia coli* cells that contain a pBluescript plasmid that contains the cDNA sequence mentioned in SEQ.ID.No.: 2, identified as cd29-4.2, has been deposited in the Spanish Type Culture Collection (CECT), University of Valencia, Edificio de Investigacion, Campus de Burjassot, 46100 Burjasot, Valencia, Spain, on the 15^{th} of July of 1999, and has been given deposit number CECT 5185.

## Claims

1. A protein based on porcine CD29 which comprises an amino acid sequence selected between:
a) the amino acid sequence shown in SEQ. ID. No.: 1, and
b) a sequence of amino acids substantially homologous and functionally equivalent to the amino acid sequence shown in SEQ. ID. No.: 1.

2. A protein according to claim 1, which has the amino acid sequence shown in SEQ. ID. No.: 1.

3. A DNA molecule which comprises a sequence that encodes a protein according to any of claims 1 or 2.

4. A DNA molecule according to claim 3, which comprises a nucleotide sequence selected among:
a) a DNA sequence identified as SEQ. ID. No.: 2; and
b) a DNA sequence analogous to the sequence defined in a) that (i) is substantially homologous to the DNA sequence defined in a); and/or (ii) codes for a polypeptide that is substantially homologous to a protein encoded by the DNA sequence defined in a).

5. A DNA molecule according to claim 4, constituted by the DNA sequence identified as SEQ. ID. No.: 2.

6. A DNA molecule according to claim 3, selected from among genomic DNA (gDNA) and copy DNA (cDNA).

7. A DNA molecule according to claim 3, that comprises a DNA sequence that encodes a porcine CD29 protein with an equal, reduced or zero expression of the Gal-α-(1,3)-Gal epitope.

8. A vector that comprises a DNA molecule according to any of claims 3 to 7.

9. A cell that comprises a DNA molecule according to any of claims 3 to 7, or a vector according to claim 8.

10. A cell according to claim 9, that is capable of glycosilating the protein expressed by said DNA molecule.

11. A polypeptide that comprises between 5 and 300 consecutive amino acids contained in the amino acid sequence of a protein according to any of claims 1 or 2.

12. A polypeptide according to claim 11, in which said consecutive amino acids are contained in the region constituted by the first 220 amino acids of the amino terminal end of the protein the amino acid sequence of which is shown in SEQ. ID. No.: 1.

13. A polypeptide according to claim 11, in which said consecutive amino acids are contained in the region constituted by the last 300 amino acids of the carboxyl terminal end of the protein the amino acid sequence of which is shown in SEQ. ID. No.: 1.

14. A monoclonal or polyclonal antibody that recognises a protein according to any of claims 1 or 2, or a polypeptide according to any of claims 11 to 13.

15. A method for producing a protein according to any of claims 1 or 2, which comprises culturing a cell according to any of claims 9 or 10 under conditions that permit the production of said protein and its recovery from the culture medium.

16. A transgenic animal that has transgenic biological material containing a DNA molecule according to claim 7.

17. Biological material selected from cells, organs and tissues of a transgenic animal according to claim 16.

18. Use of biological material according to claim 17 as xenograft in xenotransplant therapy.

19. A method for removing, *in vitro,* human natural xenoreactive antibodies that recognise a protein according to any of claims 1 or 2, that comprises putting said protein in contact with said antibodies under conditions that allow the formation of antigen-antibody complexes, and the removal of the complexes formed.

20. Method according to claim 19, in which said protein is fixed to a suitable column for affinity chromatography, and whereby the blood or serum of an individual is passed over said column under conditions that allow the formation of an antigen-antibody complex.

21. A method for removing, *in vivo,* human natural xenoreactive antibodies that recognise a protein according to any of claims 1 or 2, that comprises administering said protein, or a pharmaceutical composition that contains said protein, to the receiver of a xenograft, before the reception of said xenograft.

22. A method according to claim 21, in which said protein is administered in the form of a pharmaceutical composition suitable for parenteral administration.
